# EUROPEAN PATENT SPECIFICATION

(11) **EP 3 197 274 B1**
(45) Date of publication and mention of the grant of the patent: **18.09.2019**
(21) Application number: 15845073.4
(22) Date of filing: 01.04.2015
(51) Int. Cl.: A01N 25/10, A61L 15/22, A61L 15/24, A61L 15/26, A61L 15/28, A61L 15/44, A61L 15/46, C08L 29/04, C08L 3/02, C08L 31/04, A61F 13/84

(54) **THERMOPLASTIC ARTICLE WITH ACTIVE AGENT**
THERMOPLASTISCHER ARTIKEL MIT EINEM WIRKSTOFF
ARTICLE THERMOPLASTIQUE PRÉSENTANT UN AGENT ACTIF

(30) Priority: 26.09.2014 WO PCT/US2014/057798
(43) Date of publication of application: 02.08.2017
(73) Proprietor: Kimberly-Clark Worldwide, Inc., Neenah, Wisconsin 54956 (US)
(72) Inventor: ZHUANG, Shiming, Menasha, Wisconsin 54952 (US); VONGSA, Rebecca A., Neenah, Wisconsin 54956 (US); ZHOU, Peiguang, Neenah, Wisconsin 54956 (US); LONG, Andrew Mark, Neenah, Wisconsin 54956 (US); KOENIG, David William, Neenah, Wisconsin 54956 (US); TYRRELL, David J., Milwaukee, Wisconsin 53210 (US); ANUNSON, Paige N., Neenah, Wisconsin 54956 (US); KROLL, Lisa, Appleton, Wisconsin 54911 (US)
(74) Representative: Dehns
(86) International application number: PCT/US2015/023866
(87) International publication number: WO 2016/048413

(56) References cited:
- WO-A1-2006/042364
- WO-A2-2010/001269
- US-A1- 2007 281 003
- US-A1- 2008 200 890
- US-A1- 2013 034 719
- US-B1- 6 375 963
- US-B1- 6 375 963
- US-B2- 7 154 018
- US-B2- 7 485 110
- US-B2- 8 217 220
- US-B2- 8 552 251

## Description

### BACKGROUND

Active agents, such as antimicrobial agents or antifungal agents, have been used in conjunction with many products. In particular, since viral outbreaks such as SARS, bird flu and norovirus have been widely publicized, consumers have an increased interest in a wide array of products having an antimicrobial agent applied thereto; products such as wipes, shoe inserts, athletic clothing, personal care products, hospital equipment, sports equipment, etc. Products such as absorbent articles meant for bodily fluids (e.g. disposable diapers, pads and incontinence garments) may offer an odor-control benefit when an antimicrobial agent is applied thereto.

Currently, the application of an antimicrobial or antifungal agent onto a select portion of an absorbent personal-care article is achieved by applying it as a lotion, cream, or spray formulation. However, due to the application method, and/or the incompatibility and/or poor solubility of the active agent(s) in the formulation, getting effective concentrations of the active agents onto the personal care product is challenging.

There remains a need for a better way to apply an active agent such as an antimicrobial agent or antifungal agent to an absorbent personal-care product. For instance, there is a desire for an application method that does not require several additional steps in the manufacture of such a product (e.g. spraying, slot-coating, or the like). There is also a desire for a method of applying an active agent to such a product so that it demonstrates higher efficacy than is currently available.

WO2010/001269 discloses films formed from a water soluble polymer matrix within which is contained a fragrance.

US2013/0034719A discloses co-extruded films having a first polymer skin layer and a second stimulation layer which comprises a cooling agent and a polymeric binder. Suitable cooling agents include xylitol, sorbital, erythritol and urea. The polymeric binder may comprise polyvinyl alcohol.

### SUMMARY

This invention is directed to an extruded water-soluble film obtainable by extrusion at a temperature of 50 to 150°C of a homogeneous material comprising a water-soluble polymer that is polyvinyl alcohol and between 0.1% to 50% by weight of the film of an active agent, wherein the active agent is selected from Yucca spp, inulin or farnesol, and to a method of forming a water-soluble film according to claim 8.

### BRIEF DESCRIPTION OF THE DRAWINGS

The foregoing and other features and aspects of the present disclosure and the manner of attaining them will become more apparent, and the disclosure itself will be better understood by reference to the following description, appended claims and accompanying drawings, where;
FIG. 1 is a chart showing the dissolution time for a film (not claimed) formed according to the present disclosure;
FIGS. 2-4 are charts showing zone of inhibition on films (not claimed) containing various biocides of the present disclosure;
FIG. 5 is a chart showing the stress and strain properties of films (not claimed) having varying percentages of a first embodiment of an antimicrobial agent;
FIG. 6 is a chart showing the modulus and toughness of the films of FIG. 5;
FIG. 7 is a chart showing the stress and strain properties of films (not claimed) having varying percentages of a different antimicrobial agent to the films of FIG. 5;
FIG. 8 is a chart showing the modulus and toughness of the films of FIG. 7;
FIG. 9 is a chart showing the stress and strain properties of films (not claimed) having varying percentages of a different antimicrobial agent to the films of FIGS. 5 and 7;
FIG. 10 is a chart showing the modulus and toughness of the films of FIG. 9;
FIG. 11 is a side elevation of one embodiment of a laminate according to the present disclosure;
FIG. 12 is an exploded side elevation of one embodiment of a personal absorbent article;
FIG. 13 is a side cross-sectional view of one embodiment of an absorbent article according to the present disclosure;
FIG. 14 is a schematic showing various steps of a zone of inhibition test according to the disclosure; and
FIG. 15 is a schematic showing how test material is spread onto a medium in the test of FIG. 14;
FIG. 16 is a chart showing how inulin enhances *Lactobacillus* growth;
FIG. 17 is a chart showing a burst release of active agent; and
FIGS. 18 and 19 are charts showing a sustained release of active agent.

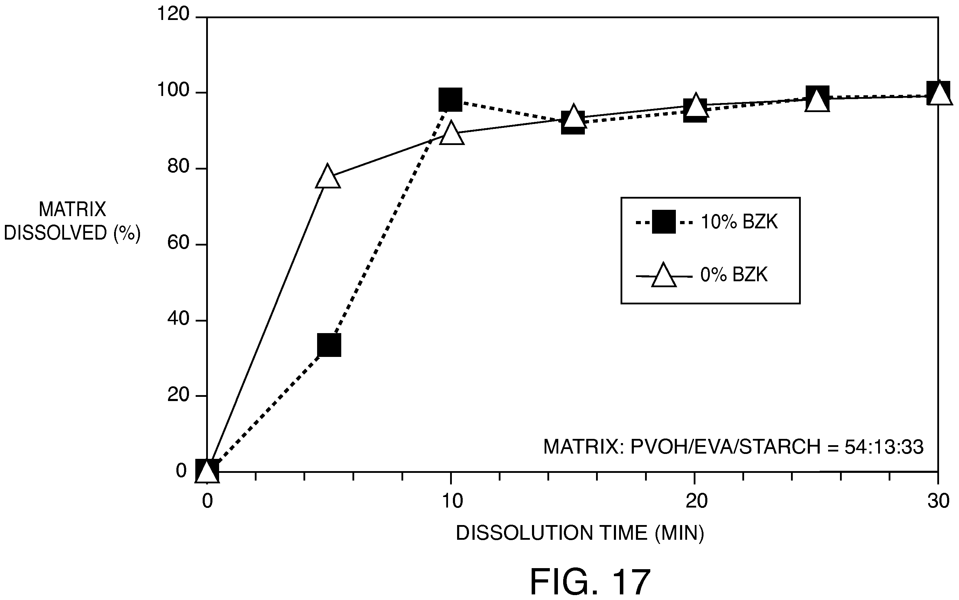

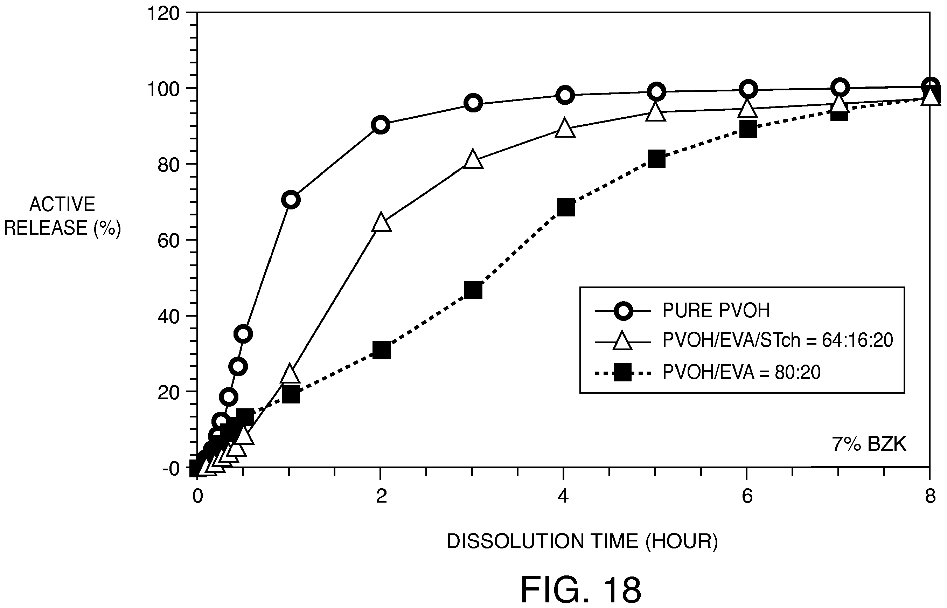

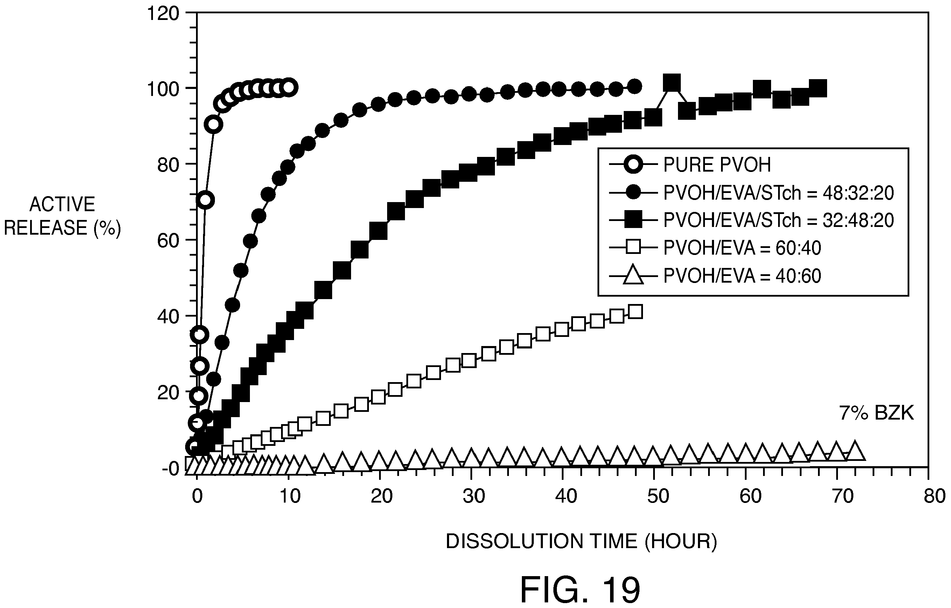

The films for which burst release of an active agent is shown in FIG. 17 and sustained release of an active agent is shown in FIGS. 18 and 19 are not claimed.

### DETAILED DESCRIPTION

It is to be understood by one of ordinary skill in the art that the present disclosure is a description of exemplary aspects of the present invention only, and is not intended as limiting the broader aspects of the present invention.

The term "laminate" refers to a material where a film structure is adhesively or non-adhesively bonded to a web such as a nonwoven or tissue material.

The term "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. In the particular case of a coform process, the meltblown fiber stream intersects with one or more material streams that are introduced from a different direction. Thereafter, the meltblown fibers and other optional materials are carried by the high velocity gas stream and are deposited on a collecting surface. The distribution and orientation of the meltblown fibers within the formed web is dependent on the geometry and process conditions. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super-Fine Thermoplastic Fibers" by K. D. Lawrence, R. T. Lukas and J. A. Young; and U.S. Patent No. 3,849,241 to Butin et al. and U.S. Patent No. 5,350,624 to Georger et al.

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblown processes, spunbond processes, air laying processes, wet layering processes and bonded-carded-web processes.

The term "personal care absorbent articles" or "absorbent articles" in the context of this disclosure includes, but is not limited to, diapers, diaper pants, training pants, absorbent underpants, incontinence products, and urinary shields; and the like.

The terms "spunbond" and "spunbond fiber" refer to fibers which are formed by extruding filaments of molten thermoplastic material from a plurality of fine, usually circular, capillaries of a spinneret, and then rapidly reducing the diameter of the extruded filaments.

The term "% by weight," "weight %," "wt%" or derivative thereof, when used herein, is to be interpreted as based on the dry weight, unless otherwise specified.

These terms may be defined with additional language in the remaining portions of the specification.

The present invention is directed to a water-soluble, thermoplastic obtainable by extrusion into which an active agent has been incorporated as defined in claim 1.

### Materials

The extruded water-soluble films now claimed include a water-soluble polymer that is polyvinyl alcohol and an active agent selected from Yucca spp, inulin or farnesol. Other optional materials that improve the performance, look, feel and/or durability may be added to the films.

**Polymer:** The water-soluble polymer used in the present invention is polyvinyl alcohol (PVOH). The film now claimed is obtainable by extrusion of a homogeneous material comprising this polymer at a temperature of 50 to 150°C. The homogeneous material may also be extruded at a temperature of 90°C to 150°C.

One desirable polymer is a highly amorphous vinyl alcohol polymer, sold as "NICHIGO G-POLYMER," available from Soarus L.L.C., Arlington Heights, Illinois. This particular polymer has a molecular weight of 10,000 to 50,000, and a relatively low crystallinity of 5 to 25%.

In one aspect, a copolymer such as ethylene vinyl acetate (EVA) may be combined with the base polymer. It is contemplated that the film of the present invention may include up to 30% by weight EVA. EVA aids in extrusion process, provides a means to control the water dissolution speed, and lowers the overall cost of the extruded material.

**Active Agent:** The active agent is selected from Yucca spp, inulin or farnesol.

Farnesol is a botanical oil. Without being bound by theory, it is believed that farnesol works by quorum sensing control and targets yeast. One of the many benefits of farnesol is that it provides a no-kill virulence control and it comes from a botanical source.

Another suitable active agent is a botanical extract that is a Yucca species extract. The Yucca species extract ("Yucca") is a phenolic. Without being bound by theory, it is believed that Yucca is a membrane uncoupler and enzyme inhibitor. Yucca is a pathogen-selective antifungal agent that can be incorporated directly into the film of the present disclosure. One particular Yucca species is Yucca schidigera which is a highly effective urease inhibitor (i.e., substances which inhibit production of ammonia from urine) when applied directly to the skin. One source of Yucca schidigera powder (100-percent pure) is sold under the trade designation DESERT PURE YUCCA by Sher-Mar Enterprises of San Diego, Ca. Another source of Yucca schidigera powder is sold under the trade designation DINASE-30-DRY by Dinatec, Inc. of Gainesville, Ga. Information relating to the efficacy of Yucca schidigera as a selective antimicrobial may be found in U. S. Patent No. 7,485,110.

For aesthetic purposes, colorants may be incorporated into articles containing Yucca to mask the relatively yellow and dark color associated with bioactive levels of Yucca. One approach for masking the color of Yucca is to co-extrude the film containing Yucca as a core layer with (a) skin layer(s) on one side, or (b) skin layer(s) on both sides. In this case, the skin layer(s) contain pigments. For example, the yellow/dark color of yucca film can be turned to white by adding suitable pigments into the skin layer(s). To prepare a white skin, about 3 to 4 percent by weight of the skin layer may be a titanium dioxide additive. To tint the white skin a color, (e.g. purple, blue, or other color) pigment may be added to the titanium dioxide additive.

Prebiotics are not antimicrobial in the sense that they kill a specific pathogen, but instead they improve the growth of healthy bacteria such as *Bifidobacterium* spp. or *Lactobacillus* spp. without promoting growth of enteropathogenic bacteria.

In one embodiment, the active agent comprises one or more inulins which are prebiotics. Inulins are generally fructose-containing oligosaccharides and belong to a class of carbohydrates known as fructans. Inulins are especially useful to promote vulval/vaginal health as they provide no-kill control of the microbiome. Inulins comprise fructose units in a beta-(2-I) glucosidic linkage and comprise a terminal glucose unit. The average degree of polymerisation of inulins generally ranges from about 10 to 12.

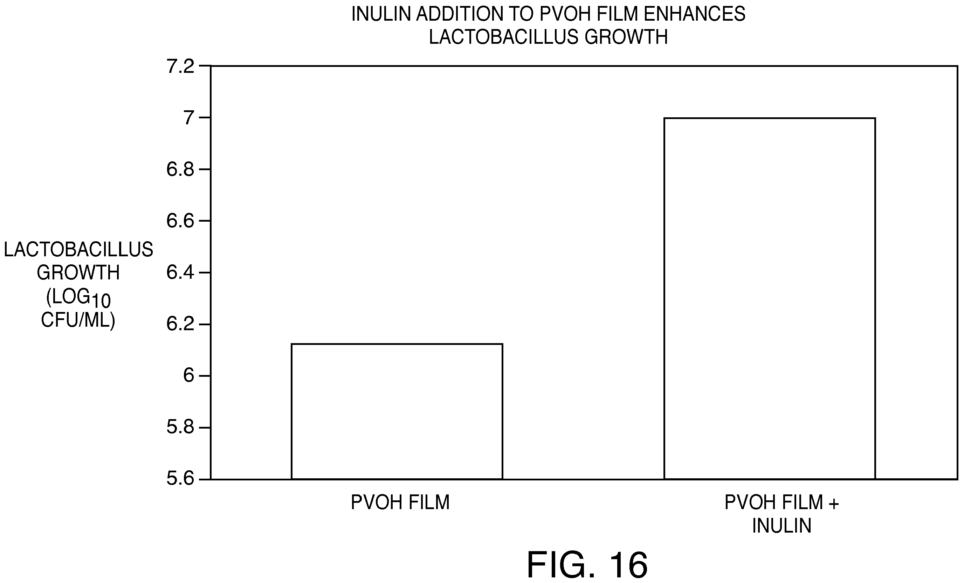

Inulins stimulate the growth of *Bifidobacterium* spp. or *Lactobacillus* spp. Referring to **FIG. 16**, demonstrated is how PVOH releases glucose, starch, and inulin, providing for an increase in growth of Lactobacillus spp. as compared to a PVOH film without an added carbon source. Pure PVOH base film causes about 0.6 log value growth of lactobacillus, which may come from the modifiers in the polymer. To highlight the effect of inulin, the reference/base-line from the PBS control shifts to the PVOH film. The pure contribution from the inulin is almost 0.9 log which indicates that the delivery approach is very effective.

The amount of active agent that is loaded into the film is limited due to the integrity of the resulting article structure. If there is too much active agent in an article, it may be unduly weakened. In one aspect, the sum of the active agent(s) is present in a total amount of 0.1% to 50% by weight of the film, or a total amount of 1% to 20% by weight of the film. In another aspect, the sum of the active agent(s) is present in a total amount of 2% to 10% by weight of the film.

**Optional Materials:** Besides the components noted above, still other additives may also be incorporated into the composition, such as fragrances, melt stabilizers, dispersion aids (e.g., surfactants), processing stabilizers, heat stabilizers, light stabilizers, UV stabilizers, antioxidants, heat aging stabilizers, whitening agents, antiblocking agents, antistatic agents, bonding agents, lubricants, colorants, etc.

In one aspect of the present invention, the extruded water-soluble film includes up to 50% thermoplastic starch by weight. The starch acts as a filler to reduce the overall cost of the extruded film. The extruded film may contain as much as 30% starch. One desirable water-soluble thermal starch is a cellulose-based starch obtained from various plant sources, hemicelluloses, modified cellulose (hydroxylalkyl cellulose, cellulose ethers, cellulose esters, etc.), and the like. When a starch is employed, the amount of such additional material may range from about 0.1 wt. % to 50 wt. % of the homogeneous blend, in some embodiments from about 0.5 wt. % to about 40 wt. %, and in some embodiments, from about 1 wt. % to about 30 wt. %.

Dispersion aids may also be employed to help create a uniform dispersion of the active agent/plasticizer. When employed, the dispersion aid(s) typically constitute from about 0.01 wt. % to about 10 wt. % of the homogeneous blend, in some embodiments from about 0.1 wt. % to about 5 wt. %, and in some embodiments, from about 0.5 wt. % to about 4 wt. %.

The composition may also contain a preservative or preservative system to inhibit the growth of microorganisms over an extended period of time. Suitable preservatives may include, for instance, alkanols, disodium EDTA (ethylenediamine tetraacetate), EDTA salts, EDTA fatty acid conjugates, isothiazolinone, benzoic esters (parabens) (e.g., methylparaben, propylparaben, butylparaben, ethylparaben, isopropylparaben, isobutylparaben, benzylparaben, sodium methylparaben, and sodium propylparaben), benzoic acid, propylene glycols, sorbates, urea derivatives (e.g., diazolindinyl urea), and so forth. Other suitable preservatives include those sold by Sutton Labs, such as "Germall 115" (amidazoiidinyl urea), "Germall II" (diazolidinyl urea), and "Germall Plus" (diazolidinyl urea and iodopropynyl butylcarbonate). Another suitable preservative is Kathon CG.RTM., which is a mixture of methylchloroisothiazolinone and methylisothiazoiinone available from Rohm & Haas; Mackstat H 66 (available from Mclntyre Group, Chicago, III.). Still another suitable preservative system is a combination of 56% propylene glycol, 30% diazolidinyl urea, 11% methylparaben, and 3% propylparaben available under the name GERMABEN.RTM. II from International Specialty Products of Wayne, N.J.

To better enhance the benefits to consumers, other optional ingredients may also be used. For instance, some classes of ingredients that may be used include, but are not limited to: antioxidants (for product integrity); astringents-cosmetic (for inducing a tingling sensation on skin); colorants (for imparting color to the product); deodorants (for reducing or eliminating unpleasant odor and protect against the formation of malodor on body surfaces); fragrances (for consumer appeal); skin conditioning agents; and skin protectants (a drug product which protects injured or exposed skin or mucous membrane surface from harmful or annoying stimuli).

### Method of Manufacture

A method of forming a water-soluble films as defined in claim 8 is also claimed.

The method includes the following steps. First, a homogenous blend is formed by combining the water-soluble polymer that is polyvinyl alcohol with at least one active agent as defined in claim 8 and optionally, one or more of the optional ingredients described herein. In one desired embodiment, the polymer is an amorphous, water-soluble polyvinyl alcohol as described herein. Second, the homogeneous blend is extruded to form a film at a temperature of from 50 to 150°C.

The homogeneous blend may be extruded at a temperature of 50°C to 125°C, or possibly 90°C to 125°C. This low extrusion-temperature profile is desirable because some active agents of interest have poor thermal stability.

Exemplary manufacturing equipment, a method of making articles, and exemplary articles are described herein.

**Extrusion Method:** The composition of the present disclosure is formed by processing the components together in a melt-blending device (e.g., extruder). The mechanical shear and heat provided by the device allows the components to be blended together in a highly efficient manner without the use of a solvent. Batch and/or continuous melt blending techniques may be employed in the present invention. For example, a mixer/kneader, Banbury mixer, Farrel continuous mixer, single-screw extruder, twin-screw extruder, roll mill, etc., may be utilized. One particularly suitable melt-blending device is a twin-screw extruder (e.g., PRISM USALAB x16, available from Thermo Electric Co., Inc., New Jersey).

The polymer and the active agent(s), along with any optional ingredients, form a homogeneous blend. For example, the materials may be blended at a shear/pressure and temperature sufficient to ensure adequate mixing (e.g., at or above the softening point of the polymer), but without adversely impacting the physical properties of the active agent. For example, melt-blending typically occurs at a temperature of from about 50°C to about 150°C., in some embodiments from about 90°C to about 130°C, and in some embodiments from about 110°C to about 125°C. These lower processing temperatures prevent degradation of the active agent.

Once formed, the homogeneous blend is extruded to create a film.

Films: In the method now claimed, the homogeneous blend is formed into a film by extrusion, either alone or in conjunction with an additional film-forming material. The film may be used in a wide variety of applications, such as a carrier of active agents for medical products, garments, absorbent articles, etc. The film may have a mono- or multi-layer configuration. Any known technique may be used to form a film by extrusion from the compounded material such as extrusion coating or coextrusion of the layers.

The process to make the antimicrobial reservoir film is relatively fast considering the high amounts of active agent that can be added to the extrusion process. In one particular embodiment, the film may be formed by flat die extrusion technique. Processes for producing such extrusions are described, for instance, in U.S. Pat. No. 7,666,337 to Yang et al.; U.S. Pat. No. 5,091,228 to Fuji et al; and U.S. Pat. No. 4,136,145 to Fuchs et al.

The film may be optionally oriented in one or more directions to further improve film uniformity and reduce thickness. For example, the film may be immediately reheated to a temperature below the melting point of one or more polymers in the film, but high enough to enable the composition to be drawn or stretched. In the case of sequential orientation, the "softened" film is drawn by rolls rotating at different speeds or rates of rotation such that the sheet is stretched to the desired draw ratio in the longitudinal direction (machine direction). The film may be made into thicknesses ranging from 0.01 mm up to about 1 mm, or in other aspects, from 0.05 mm to 0.20 mm.

The multi-layer film may contain from two (2) to nine (9) layers, and in some embodiments from three (3) to five (5) layers. In one example, the multi-layer film has one base layer and one skin layer. The base layer and/or skin layer may contain the active agent(s). The ratio between the layers may range from 1 to 20.

In another example, there is a three-layered film having a core layer "C" that contains an active agent as described herein. The outer skin layers "S" may act as a protective layer to the core. The ratio between the layers may range from 2% to 98% of the core layer and from 10% to 90% of the two combined skin layers. For instance, the core layer may be up to about 30%, up to about 40%, up to about 50%, up to about 60%, or up to about 70% of the total thickness of the multi-layer film. Each skin layer may be up to about 15%, or up to about 25%, or up to about 35% of the total thickness of the multi-layer film.

The film, either mono- or multi-layered, may be wound and stored on a take-up roll. Various additional potential processing and/or finishing steps known in the art, such as slitting, treating, aperturing, printing graphics may be performed.

In one aspect, the extruded water-soluble film has a basis weight of 5 gsm to 500 gsm. In another aspect, the water-soluble film has a basis weight of 20 gsm to 200 gsm.

In one aspect, the extruded water-soluble film has a tensile strength of 0.5 MPa to 50 MPa according to the Tensile Test of the present disclosure. In another aspect, the film has a tensile strength of 1 MPa to 25 MPa according to the same test.

In one aspect, the extruded water-soluble film has a water dissolution speed from 5 seconds to 30 minutes as determined by the Water Dissolution Test of the present disclosure. In another aspect, the extruded water-soluble article film has a water dissolution speed of 30 seconds to 5 minutes as determined by the same test.

In one aspect, the extruded water-soluble film has an elongation of 5% to 500% according to the Tensile Test of the present disclosure. In another aspect, the film has an elongation of 10% to 100% according to the same test.

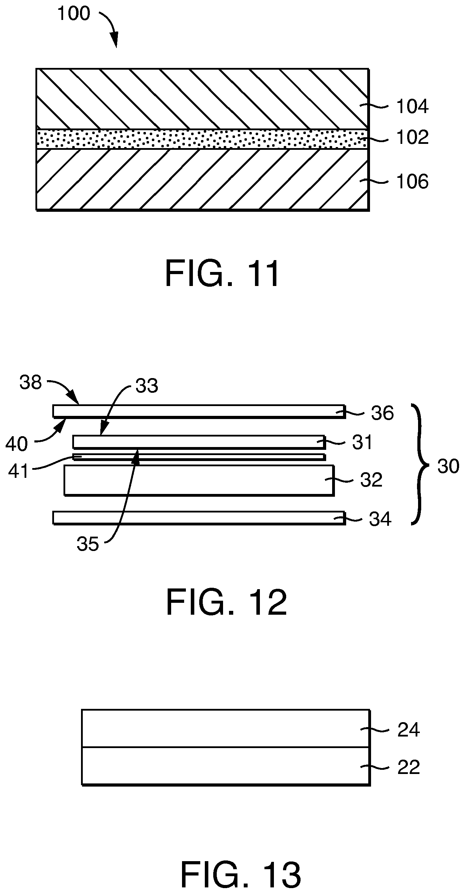

Referring to **Fig. 13**, a laminate may be formed by extruding the homogeneous blend 24 onto a carrier substrate 22, forming a bond therebetween. The carrier substrate 22 may be a nonwoven or woven material. The laminate may also be formed by adhering the film of the present disclosure to a substrate using an adhesive. Suitable adhesives include polyolefin-based hot melt construction and elastic adhesives. Examples of suitable materials include hydrophobic and hydrophilic hot melt polymers, such as those available from Henkel (having a place of business located in Bridgewater, New Jersey, U.S.A.) such as 34-5610, 34-447A, 70-3998 and 33-2058; those available from Bostik-Findley (having a place of business located in Milwaukee, Wisconsin, U.S.A.) such as HX 4207-01, HX 2773-01, H2525A, H2800, H9574; and those available from H.B. Fuller Adhesives (having a place of business located in Saint Paul, Minnesota, U.S.A.) such as HL8151-XZP. Other adhesives are further described in U.S. Patent Publication No. 2005/0096623 to Sawyer, et al.

### Applications

**Absorbent Articles:** The film of the present invention is particularly suitable for use in an absorbent article. An "absorbent article" generally refers to any article capable of absorbing water or other fluids. Examples of some absorbent articles include, but are not limited to, personal care absorbent articles, such as diapers, training pants, absorbent underpants, incontinence articles, feminine hygiene products (e.g., sanitary napkins, pantiliners, etc.), swim wear, baby wipes, and so forth; medical absorbent articles, such as garments, fenestration materials, underpads, bedpads, bandages, absorbent drapes, and medical wipes; food service wipers; clothing articles; and so forth. Several examples of such absorbent articles are described in U.S. Patent Nos. 5,649,916 to DiPalma, et al.; 6,110,158 to Kielpikowski; 6,663,611 to Blaney, et al. Still other suitable articles are described in U.S. Patent Application Publication No. 2004/0060112 A1 to Fell et al., as well as U.S. Patent Nos. 4,886,512 to Damico et al.; 5,558,659 to Sherrod et al.; 6,888,044 to Fell et al.; and 6,511,465 to Freiburger et al. Materials and processes suitable for forming such absorbent articles are well known to those skilled in the art.

The present disclosure may be better understood with reference to the examples presented herein.

First Exemplary Absorbent Article: Referring to **FIG. 12**, in one aspect of the disclosure, a personal absorbent article 30 includes an absorbent member 32 sandwiched between a water-impermeable backsheet 34 and a water-permeable liner 36, wherein liner 36 has a body-facing surface 38 and an opposite outward-facing surface 40. A film 41 of the present invention is attached to either the outward-facing surface 40 of the liner or a surface of the absorbent member 32 that is adjacent liner 36. Desirably, film 41 is in direct contact with liner 36. Should a multi-layer film be used for film 41, the layer containing the largest amount of active agent is adjacent liner 36 so that the active agent can more easily leach through the liner to contact the wearer's body.

Film 41 is made from materials as defined in claim 1, is obtainable by extrusion at a temperature of 90 to 150°C and additionally comprises a plasticizer.

Second Exemplary Absorbent Article: Referring to **FIG. 11**, in one aspect, the film of the present invention is laminated to other layers (e.g., nonwoven or cellulose-fiber based web materials). One particular application of a laminate structure is that of a three-layer wipe 100. In this embodiment, the core layer 102 is a film containing at least the active agent(s) of the present disclosure. Desirably, the outer layers 104 and 106 that surround the core layer are natural or synthetic fiber based web materials (e.g. tissue, paper, spunbond, spunbond-meltblown-spunbond composite, coform, airlaid, etc.). Other uses for laminates are contemplated, such as using the laminate to manufacture garments such as disposable lab coats or disposable booties. Another application may be to attach a piece of a laminate onto a bathing sponge for imparting the active agents of the disclosure to the skin while bathing.

**Burst or Sustained Release Films:** Referring now to **FIG. 17**, shown is a release profile for a BZK film (film not claimed) having the following matrix: PVOH/EVA/Starch=54:13:33. It is a "burst release" because it releases the active agent in about 1 to 3 minutes. It takes about 10 minutes for 100 percent of the active to be released from the film. Sustained release films can be created by varying the components of the film. Referring now to **FIG. 18**, shown is a sustained release profile for BZK films (not claimed) having varied matrixes: 100 percent PVOH; PVOH/EVA=80:20; and PVOH/EVA/Starch=64:16:20. Each film contains 7 percent BZK by weight. For the pure PVOH film, a release of 90 percent BZK takes about 2 hours. Blending 20 percent EVA with the PVOH lowers the release rate of BZK from the matrix: the time to release 90 percent of the BZK is about 7 hours. Adding 20 percent starch to the EVA/PVOH blend speeds the release rate of BZK to about 4 to 5 hours. Referring now to **FIG. 19**, shown is a sustained release profile for BZK films (not claimed) having varied matrixes: 100 percent PVOH; PVOH/EVA/Starch=48:32:20; PVOH/EVA/Starch=32:48:20; PVOH/EVA=60:40; and PVOH/EVA=40:60. Each film contains 7 percent BZK by weight. For the pure PVOH film, a release of 100 percent BZK takes about 5 hours. For the PVOH/EVA/Starch=48:32:20 film, a release of 100 percent BZK takes about 35 hours. For the PVOH/EVA/Starch=32:48:20 film, a release of 100 percent BZK takes about 65 hours. For the PVOH/EVA=60:40 film, a release of 40 percent BZK takes about 48 hours. Finally, for the PVOH/EVA=40:60 film, a release of less than 5 percent BZK takes more than 70 hours.

### Experimental Data

Experiment 1 (reference): Provided is experimental data for three antimicrobial agents that act as biocides, namely, zeolite, benzoalkonium chloride, and didecyl dimethyl ammonium chloride. Tests were performed on the various codes for each biocide to determine dissolution, zone of inhibition, and mechanical properties.

**TABLE 1**

| | | **Additives** | |
|---|---|---|---|
| **Category** | **Code** | **%** | |
| **Antimicrobial Agents** | GP25-C00 | 0 | Inorganic biocide (zeolite) Sourced from: Jishim Tech Co., Lid (Korea), CWT-A (brand name). |
| | GP25-C05 | 5 | |
| | GP25-C10 | 10 | |
| | GP25-C20 | 20 | |
| | GP25-C50 | 50 | |
| | GP25-B01 | 1 | BZK (benzoalkonium chloride) Sourced from Mason Chemical, Company, IL, under NOBAC (brand name). |
| | GP25-B02 | 2 | |
| | GP25-B05 | 5 | |
| | GP25-B10 | 10 | |
| | GP25-D01 | 1 | DDAC (didecyl dimethyl ammonium chloride) Sourced from, Lonza Inc, NJ, BARDAC 2250 (brand name). |
| | GP25-D02 | 2 | |
| | GP25-D05 | 5 | |
| | GP25-D10 | 10 | |

Films (not claimed) containing the various amounts of the antimicrobial agents of Table 1 were made as follows. A twin-screw extruder (PRISM USALAB x16, available from Thermo Electric Co., Inc.) was used to make co-extruded film samples that contain an antimicrobial agent. The extruder specifications were as follows:
- 16 mm diameter screw
- L/D=40 (L=640 mm)
- 10 heating zones + die
- Maximum velocity = 1000 rpm
- Maximum pressure = 100bar [10 MPa]
- Maximum torque = 24 mN

The following extruder set-up was used to manufacture the experimental film:
- Flat slit die width: 152.40mm (6")
- Flat slit die height (controls film thickness): 0.127mm (0.010")

Each coextruded film included one of the active ingredients of Table 1.

The extruder feed zone was heated to 110°C, the following extruder zones 2-9 were heated to 125°C, and the die was heated to 130°C. The material was extruded.

### Dissolution Test:

### I. Preparation of specimens:

a. Cut 9 film specimens (approximately 0.75" x 2.5" [1.9 x 6.4 cm] or 0.07-0.12 g each). Record the mass of each specimen.
b. Match each specimen with a tall 2 oz [57g] glass jar and lid. Fill each jar with enough buffered water so that the water is 100 times the weight of the film. Three jars are to be filled with a pH 5 buffered solution, three with a pH 7, and 3 with a pH 9 buffered solution.
   i. The buffered solutions contain:
      1. pH 5: 990 g tap water, 10 g sodium citrate, 1.89 g citric acid
      2. pH 7: 990 g tap water, 10 g sodium citrate, 0.18 g citric acid
      3. pH 9: 990 g tap water, 10 g sodium citrate, 1.02 g triethanolamine
c. Heat one jar from each pH to 60°C, and one jar from each pH to 40°C. The last jar of each pH remains at room temperature (approx. 20°C)

### II. Testing of specimens:

a. Gather the film specimens, a stopwatch, a glass stir-rod, and the jars of buffered water.
b. Drop a film specimen into each jar, using the glass rod to submerge the film specimen if necessary. Do not drop the sample onto the wall of the jar, as the film will adhere and take longer to dissolve.
c. Start the timer immediately after submerging the film specimen.
d. Record the time that the film is 95%+ dissolved. Swirl the jar if necessary to check to see if the film is dissolved. Some films cloud the water and make it difficult to discern when the specimen is dissolved.

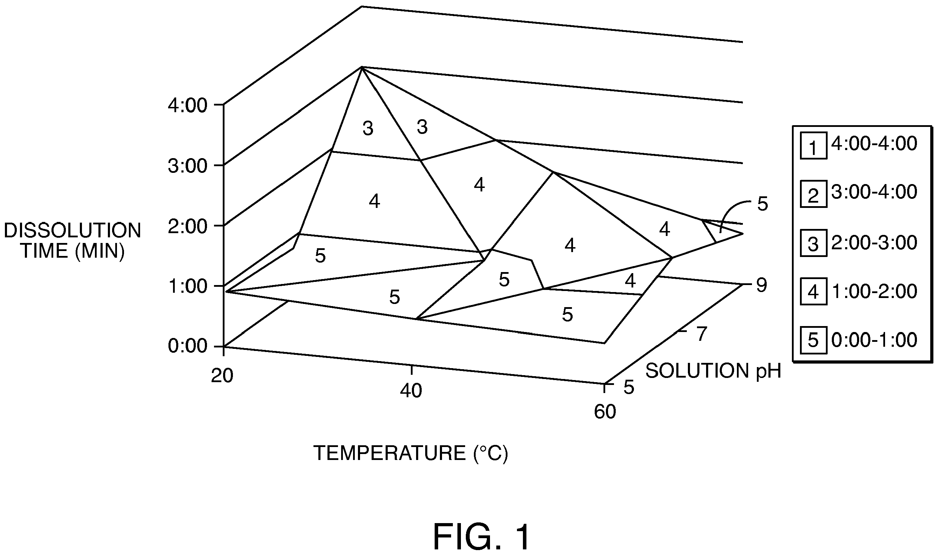

**FIG. 1** is a three-dimensional graph showing the dissolution time for antimicrobial GP25-C05, at varying pH and temperature. The longest dissolution time of three minutes is shown at a condition of pH 9 and 20°C. In contrast, the shortest dissolution time of less than 1 minute is shown at a condition of pH 5 and 60°C.

Zone of Inhibition Test: In this test method, the test material is brought into contact with a known population of microorganisms on an agar plate for a specified period of time. At the end of the contact time, the area of inhibited colony formation around the test material is measured. The size of this area of no growth is a measure of leaching of the antimicrobial agent from the test material.

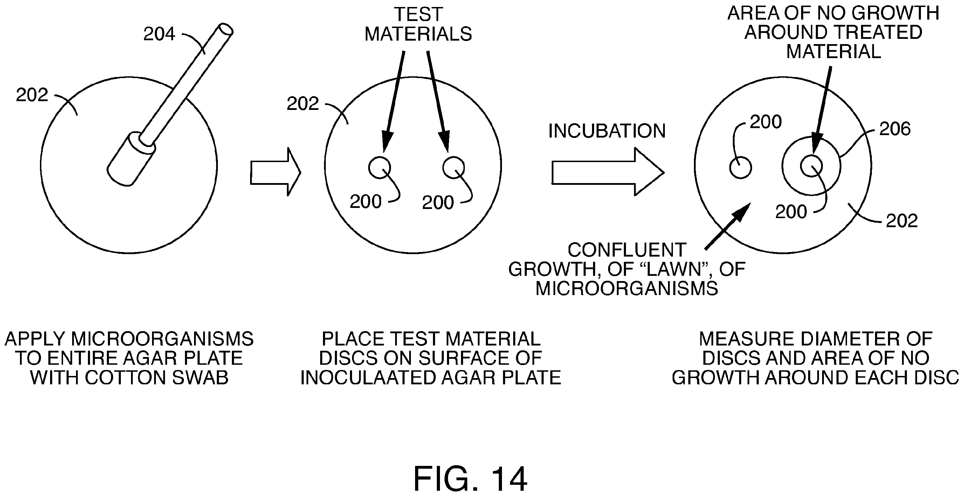

Referring to **FIG. 14**, the test material 200 is cut into small discs and placed on an agar plate 202 evenly spread with a test microorganism with a cotton swab 204. The plates are incubated for 24 hours at ideal growth conditions. Following incubation, the diameter of the circle of no growth 206 around the disc 200 is measured. The zone of inhibition is reported as the difference between the sample disc diameter and the average of the measured no growth zone diameters.

### Materials and Reagents:

- Microorganisms: frozen stock of *Staphylococcus aureus* (ATCC 27660) and Pseudomonas aeruginosa (ATCC 15442) *Staphylococcus aureus* (ATCC 6538), *Escherichia coli* (ATCC 8739), and *Candida albicans* (ATCC 10231).
- Mueller-Hinton agar (MHA) plates or equivalent plated media. Prepare following manufacturer's directions. Store at 4± 2 °C. Alternatively, pre-made plates can be utilized.
- Mueller-Hinton broth (MHB) or equivalent liquid media. Prepare following manufacturer's directions. Store at 4 ± 2 °C. Alternately, pre-made media can be utilized.
- Sterile cotton swabs or equivalent.
- Sterile forceps.
- Positive control disc: Vanocymicin susceptibility discs (6 mm), 30 µg/disc (BD and Company; Sparks, Maryland).
- Test material, cut into 8 mm discs.
- Calipers or other measuring device.
- Other ancillary lab supplies.

### Supply Set-up:

1. Label growth media plates appropriately for testing codes.
2. Sterilize test material discs with UV exposure in Laminar flow hood for 15 minutes (both sides of disc), if required.

### Inoculum:

1. Take appropriate measures to ensure culture purity.
2. Staphylococcus aureus or Pseudomonas aeruginosa is inoculated from an overnight plate or MHB into 5 ml of sterile MHB in a 35 °C incubator for 18 - 24 hrs.
3. The overnight culture is then adjusted using MHB to the 0.5 McFarland barium sulphate standards (1 x 108 CFU/ml) or approximately 0.15 OD with a 0.2 cm light path at 660 nm.
4. Discard the cell suspension if it is not used within 30 to 60 min after preparation.

### Zone of Inhibition Bioassay Procedure:

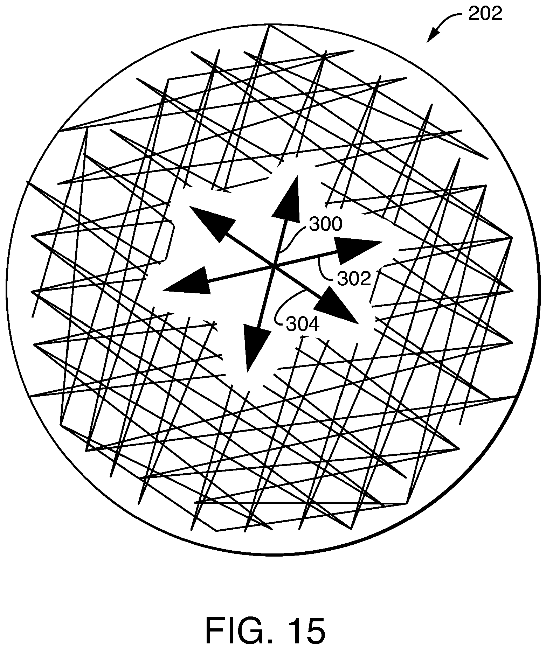

1. Pre-warm the MHA plates to room temperature. The number of plates required per strain will depend on the number of test materials to be tested and their anticipated zone inhibition diameters; discs should be placed on plates so that zones of inhibition do not overlap.
2. The surface of the plates should be dry. If not, dry the plates (with lids ajar) in a 35 °C incubator for 20 - 30 min just prior to inoculation. There should be no visible droplets of moisture on the surface of the agar or on the lids of the plates when they are inoculated.
3. Moisten a sterile applicator swab in the standardized cell suspension and express any excess moisture by rotating the swab against the glass above the liquid in the tube. Referring to FIG. 15, inoculate the entire surface of each agar plate 202, inoculating the surface completely in three different directions 300, 302, 304 to ensure uniform growth.
   (It is recommended that cotton swabs with wooden handles be used for this procedure. Swabs made of synthetic materials do not soak up sufficient suspension to inoculate the entire surface of the plate. Swabs with plastic handles bend when excess suspension is being expressed and may splatter liquid out of the tube.)
4. Repeat step 10.3 to inoculate additional plates as needed.
5. Store the inoculated plates at room temperature for 10 - 15 min to allow the medium to absorb the moisture from the inoculum.
6. Apply discs of test material to the surface of the inoculated medium with a sterile forceps and tap them to ensure that they are in complete contact with the agar surface. A positive control (vancomycin disc) and negative control (uncoated disc) should be used on each plate. All discs should be approximately the same distance from the edge of the plate and from each other (FIG. 14). In addition, all the discs should be positioned so the area of no growth that may develop around them do not overlap.
7. Invert the inoculated plates and incubate them at 35 °C for 18 - 24 hours.
8. Examine the plates from the back, viewed against a black background and illuminated with reflected light. With calipers, measure the diameter of each zone of inhibition to the nearest whole millimeter.

Calculation: The zone of inhibition is equal to the diameter of the no-growth area minus the diameter of the disc.

The inhibition zone sizes given in this test protocol are derived from test methods used at the Center for Disease Control as well as AATCC Method 147-1998 (19) based on the National Committee for Clinical Laboratory Standards (20-21) and ASTM E2149-01 step 12.2 (22). The diameters of zones of inhibition may vary depending on many factors including medium base, humidity, and the age of the medium. Thus, it is important to follow one protocol as closely as possible to obtain results comparable between labs, personnel, and experiments. It may be necessary to determine zone interpretative sizes for disc diffusion results that are appropriate to local conditions. These criteria may be determined with use of reference strains and known challenge compounds and amounts.

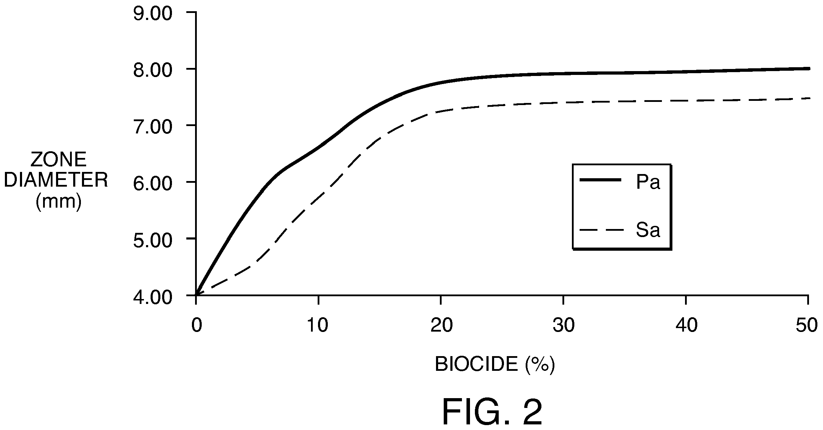

Results: **FIG. 2** shows the result of the zone of inhibition testing for films (not claimed) containing antimicrobial agent CWT-A. This agent was more effective against Pseudomonas aeruginosa (Pa) than Staphylococcus aureus (Sa), but the effectiveness against both microbes plateaued when the film contained 20% or more of the antimicrobial agent.

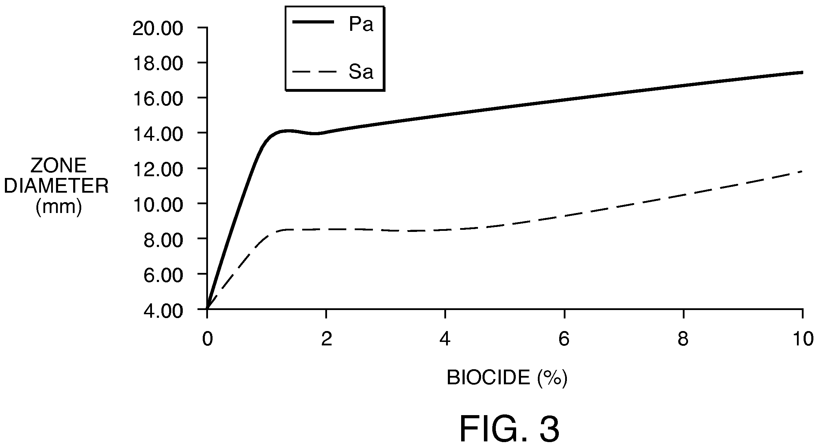

**FIG. 3** shows the result of the zone of inhibition testing for films (not claimed) containing antimicrobial agent DDAC. This agent was significantly more effective against Staphylococcus aureus (Sa) than Pseudomonas aeruginosa (Pa). The effectiveness against Sa microbes went from a zone of 4 mm to 14 mm between 0% and about 1% DDAC. When the film contained from about 1% and 10% DDAC, the zone of inhibition of the Sa microbes went from about 14 mm to about 17 mm. The effectiveness against Pa microbes went from a zone of 4 mm to about 7 mm between 0% and about 1% DDAC. When the film contained from about 1% and 10% DDAC, the zone of inhibition of the Pa microbes went from about 9 mm to about 12 mm, plateauing at about 9 mm between about 1% and 4% DDAC.

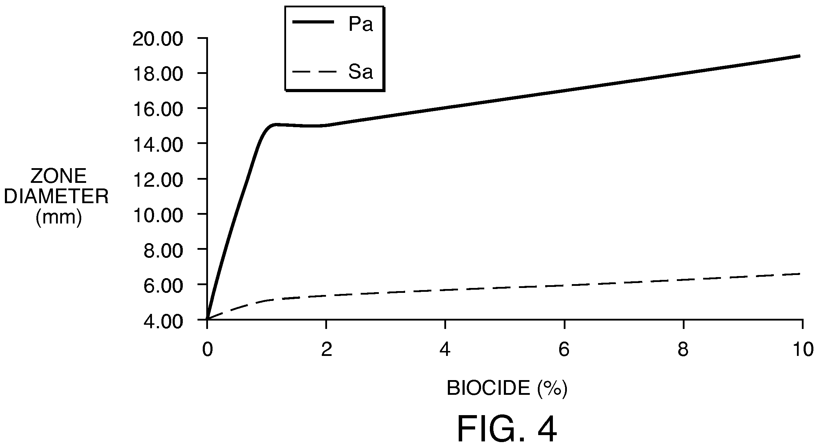

**FIG. 4** shows the result of the zone of inhibition testing for films (not claimed) containing antimicrobial agent BZK. This agent was much more effective against Staphylococcus aureus (Sa) than Pseudomonas aeruginosa (Pa). The effectiveness against Sa microbes went from a zone of 4 mm to 14 mm between 0% and about 1% BZK. When the film contained from about 1% and 10% BZK, the zone of inhibition of the Sa microbes went from about 15 mm to about 19 mm. The effectiveness against the Pa microbes went from a zone of 4 mm to about 6mm between 0% and about 10% BZK.

Tensile Test: Prior to testing, samples were initially conditioned at 75°F [24°C]/50% relative humidity for 24 hours. Thereafter, the strip tensile strength values were determined in accordance with ASTM Standard D-5034. A constant-rate-of-extension type of tensile tester was employed. The tensile testing system was a Synergie 200 tensile frame. The tensile tester was equipped with TESTWORKS 4.08B software from MTS Systems Corp. to support the testing. An appropriate load cell was selected so that the tested value fell within the range of 10-90% of the full scale load. The film samples were initially cut into dog-bone shapes with a center width of 3.0 mm before testing. The samples were held between grips having a front and back face measuring 25.4 millimeters x 76 millimeters. The grip faces were rubberized, and the longer dimension of the grip was perpendicular to the direction of pull. The grip pressure was pneumatically maintained at a pressure of 40 pounds per square inch [276 kPa]. The tensile test was run using a gauge length of 18.0 millimeters and a break sensitivity of 40%. Five samples were tested by applying the test load along the machine-direction and five samples were tested by applying the test load along the cross-direction. During the test, samples were stretched at a crosshead speed of about 127 millimeters per minute until breakage occurred. The modulus of elasticity, peak load, peak stress, elongation (percent strain at break), and energy per volume at break (total area under the stress-strain curve) were measured.

The tensile test results showed that the films have excellent mechanical properties for highspeed converting processes. This allows the films to easily be placed into articles such as the absorbent articles and laminates described herein.

### Test Results:

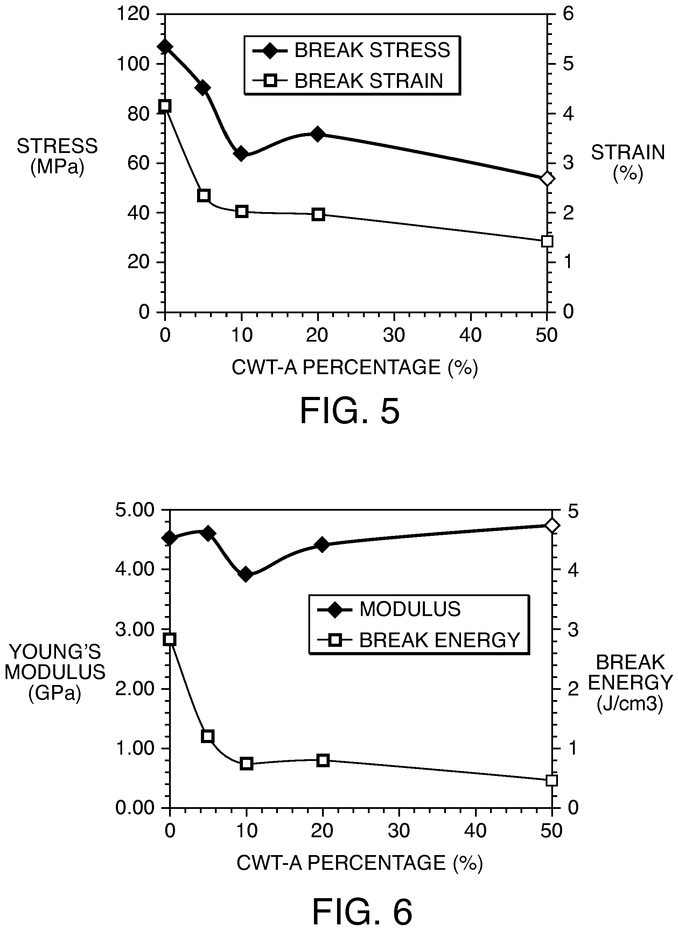

**FIGS. 5-10** show the test results from the tensile tests described above.

Referring to **FIG. 5**, a dual chart shows how the stress and strain vary with the percentage of antimicrobial in the tested film (not claimed), the antimicrobial being zeolite. The greatest break stress occurs when the film contains 0% zeolite by weight. The break stress drops rapidly as zeolite is added, and plateaus somewhat at about 10% zeolite content by weight. Like the break stress, the break strain is greatest when the film contains 0% zeolite by weight, and generally plateaus after about 7% zeolite by weight has been added.

Referring to **FIG. 6**, a dual chart shows how the elasticity and toughness vary with the percentage of antimicrobial in the tested film (not claimed), the antimicrobial being zeolite. The greatest elasticity occurs when the film contains 50% zeolite by weight. The least amount of elasticity is seen at about 10% by weight zeolite. Like the break stress, toughness is greatest when the film contains 0% zeolite by weight, and generally plateaus after about 10% zeolite by weight has been added.

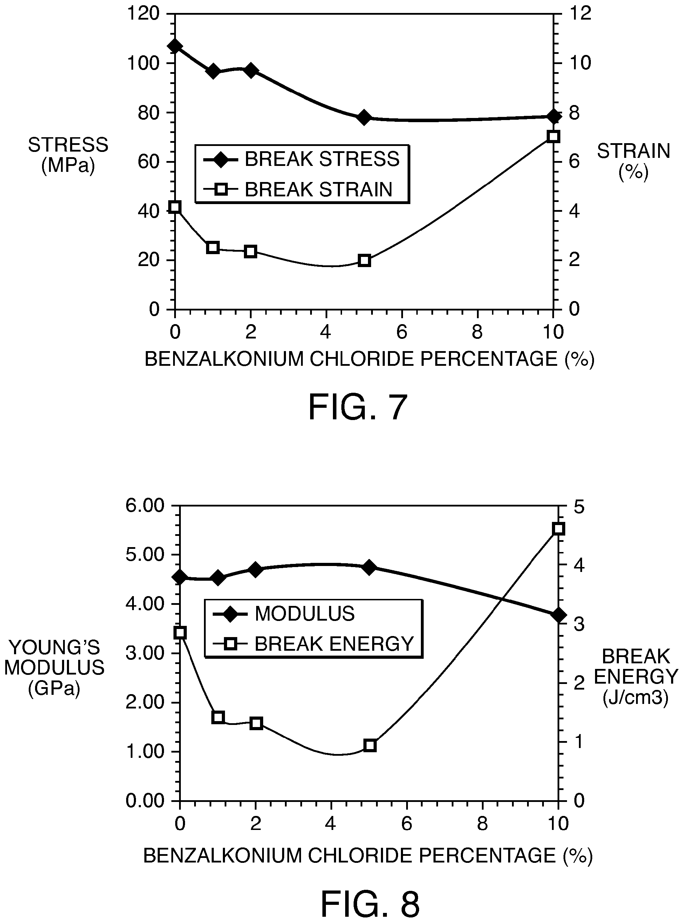

Referring to **FIG. 7**, a dual chart shows how the stress and strain vary with the percentage of antimicrobial in the tested film (not claimed), the antimicrobial being benzoalkonium chloride. The greatest break stress occurs when the film contains 0% benzoalkonium chloride by weight. The break stress drops as benzoalkonium chloride is added, and plateaus somewhat at about 5% benzoalkonium chloride content by weight. Unlike the break stress, the break strain is greatest when the film contains 10% benzoalkonium chloride by weight, and generally plateaus when between about 1% and 5% benzoalkonium chloride by weight has been added.

Referring to **FIG. 8**, a dual chart shows how the elasticity and toughness vary with the percentage of antimicrobial in the tested film (not claimed), the antimicrobial being benzoalkonium chloride. The greatest elasticity occurs when the film contains 4% benzoalkonium chloride by weight. The least amount of elasticity is seen at about 10% by weight benzoalkonium chloride. Unlike the break stress, toughness is greatest when the film contains 10% benzoalkonium chloride by weight. A sharp rise from its lowest toughness occurs at about 4% benzoalkonium chloride by weight.

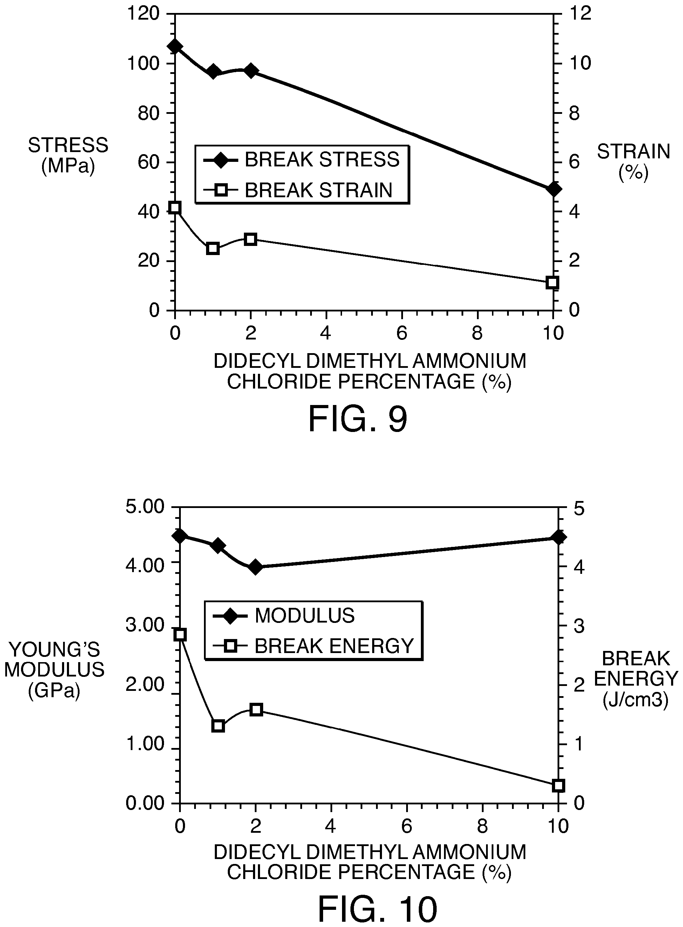

Referring to **FIG. 9**, a dual chart shows how the stress and strain vary with the percentage of antimicrobial in the tested film (not claimed), the antimicrobial being didecyl dimethyl ammonium chloride. The greatest break stress occurs when the film contains 0% didecyl dimethyl ammonium chloride by weight. The break stress drops as didecyl dimethyl ammonium chloride is added, with only a small plateau between about 1 to 2% didecyl dimethyl ammonium chloride by weight. Like the break stress, the break strain is greatest when the film contains 0% didecyl dimethyl ammonium chloride by weight, and drops somewhat steadily as it is added.

Referring to **FIG. 10**, a dual chart shows how the elasticity and toughness vary with the percentage of antimicrobial in the tested film (not claimed), the antimicrobial being didecyl dimethyl ammonium chloride. The greatest elasticity occurs when the film contains either 0% or 10% didecyl dimethyl ammonium chloride by weight. The least amount of elasticity is seen at about 2% by weight didecyl dimethyl ammonium chloride. Like the break stress, toughness is greatest when the film contains 0% didecyl dimethyl ammonium chloride by weight. TH toughness drops steadily after 2% didecyl dimethyl ammonium chloride by weight has been added to the film, following a minor plateau between the 1% and 2% didecyl dimethyl ammonium chloride by weight has been added to the film.

Experiment 2: Inulin was included in PVOH films and tested for an increase in growth of *Lactobacillus* spp. as compared to a PVOH film without an added carbon source. A 48-hour culture of *Lactobacillus acidophilus* ATCC 314 was added to a 6 well microplate containing controls and films of specified size for PVOH and PVOH + Inulin. Phosphate buffer solution and media (LAPT-Glucose) with *L. acidophilus* (∼4.5 LOG₁₀ CFU/mL) were added to wells containing PVOH films. The 6-well plate was incubated at 37°C for 24 hours, removed, and the solutions were diluted appropriately and plated. After 48 hours of incubation at 37°C, the plates were removed and counted for results. Figure 16 demonstrates how PVOH is able to release inulin and provides an increase in growth of *Lactobacillus* spp. as compared to a PVOH film without an added carbon source.

The testing results indicated that the pure PVOH base film also caused about 6.1 LOG₁₀ (CFU/mL) growth of lactobacillus, which could come from the modifiers in the G-polymer. To highlight the effect of inulin, the pure contribution from the inulin is near 0.9 LOG₁₀ (CFU/mL) growth over the PVOH film without an added carbon source, which indicates the delivery approach is very effective and the prebiotic is still active.

### Test Method

### Materials

- *Lactobacillus acidophilus* ATCC 314
- Lactobacilli MRS broth, 500g, Fischer Sci DF 0881-17-5
- Difco Lactobacilli MRS agar, 500g, Fisher DF 0882170
- Phosphate buffer (Remel with Magnesium chloride)
- Crystalgen Bio-Degradable Green Culture Flasks, 125 ml (Fisher 05-539-804)
- BD™ Difco™ Disposable Inoculating Needles 10µl; Light blue; Pack of 250 (Fisher 22-031-22)
- 15 mL centrifuge tube
- GasPack EX anaerobe sachets, Fischer B-260678

### Culture

1. Aseptically add 20 mL of MRS broth to a disposable, sterile culture flask
2. Remove test organism from -80°C
3. Using 1 (10 µL) disposable loop, remove a single bead from stock and add to the broth
4. Grow for 48 hours, 37°C, static conditions
5. Remove flask from incubator; assume starting concentration organism is 10⁷⁻⁸ CFU/mL

### Experimental

1. Centrifuge & wash 48 hour grown cells 1x with 20 ml PBS; 8,000 rpm (10,322 g) for 10 minutes at 4°C
2. Dilute 1:100 in PBS (10 µL into 990 µL)
3. Dilute 1:100 in LAPT-G media (glucose depleted) (140 µl into 13.0 mL) 19
4. Determine concentration of inoculum by plating out -1,-2,-3 on MRS agar plates, incubate anaerobically 2 days.

### Day 1a: 6-well plate set-up

1. Set-up 2 replicate plates for each code
   - Add 1.0 ml PBS to wells containing PVOH films
   - Add 1.0 ml inoculum (*L*. *acidophilus* in LAPT-G) to all wells

### Day 1b: Incubation of 6-well plate

1. Place 6-well plate and inoculation plates into anaerobic box; place plate lid slightly askew
2. Add 3 packages BD Gas Pak EZ anaerobe container system packs into box and close lid
3. Incubate 24 hours at 37± 2 °C

### Day 2: Incubation of 6-well plate

1. Dry MRS lab made plates in laminar flow cabinet 15-30 minutes
2. Prepare dilutions in PBS
3. Plate out samples and controls from 6-well plate in duplicate
4. Incubate plates 48 hours, as described above (37±2 °C, anaerobic conditions)

### Day 4: Results

1. Count MRS plates and record results.

As will be appreciated by those skilled in the art, changes and variations to the present disclosure are considered to be within the ability of those skilled in the art. Examples of such changes are contained in the patents identified above. Such changes and variations are intended by the inventors to be within the scope of the present disclosure. It is also to be understood that the scope of the present disclosure is not to be interpreted as limited to the specific aspects disclosed herein, but only in accordance with the appended claims when read in light of the foregoing disclosure.

## Claims

1. An extruded water-soluble film obtainable by extrusion at a temperature of 50 to 150°C of a homogeneous material comprising a water-soluble polymer that is polyvinyl alcohol and between 0.1% to 50% by weight of the film of an active agent, wherein the active agent is selected from Yucca spp, inulin or farnesol.

2. The extruded water-soluble film of claim 1 obtainable by extrusion at a temperature of 90°C to 125°C, wherein the polymer is an amorphous polyvinyl alcohol matrix.

3. The extruded water-soluble film of claim 1 further comprising up to 50% starch by weight.

4. The extruded water-soluble film of claim 1 further comprising up to 30% by weight of ethylene vinyl acetate (EVA).

5. The extruded water-soluble film of claim 1, wherein the film has a water dissolution speed from 5 seconds to 30 minutes as determined by a Water Dissolution Test of the present disclosure, or wherein the film has a basis weight of 5 gsm to 500 gsm; or wherein the film has a tensile strength of 0.5 MPa to 50 MPa according to a Tensile Test of the present disclosure; or wherein the film has an elongation of 2% to 200% according to a Tensile Test of the present disclosure.

6. A personal absorbent article comprising: an absorbent member disposed between a water-impermeable backsheet and a water-permeable liner, wherein the liner has a body-facing surface and an opposite garment-facing surface; and wherein the extruded water-soluble film of claim 1 is attached to the liner or a surface of the absorbent member adjacent the liner.

7. A method of forming a water-soluble film, the method comprising:
forming a homogeneous blend comprising a water-soluble polymer that is polyvinyl alcohol and at least one active agent; and
extruding the homogeneous blend to form a film at a temperature of from 50 to 150°C;
wherein the active agent is selected from Yucca spp, inulin or farnesol.

8. The method of claim 7, wherein the polymer is an amorphous polyvinyl alcohol.

9. The method of claim 7, wherein the polymer is an amorphous polyvinyl alcohol matrix and wherein the homogeneous blend is extruded at a temperature of from 90°C to 125°C.

10. The method of claim 7, wherein the homogeneous blend is extruded at a temperature of from 50°C to 125°C.

11. The method of claim 7, wherein the homogeneous blend is extruded at a temperature of from 90°C to 125°C.

## Patentansprüche

1. Extrudierte, wasserlösliche Folie, die durch Extrusion bei einer Temperatur von 50 °C bis 150 °C aus einem homogenen Material erhalten werden kann, umfassend ein wasserlösliches Polymer, das Polyvinylalkohol ist und zwischen 0,1 Gewichts-% und 50 Gewichts-% der Folie aus einem Wirkstoff besteht, wobei der Wirkstoff aus Yucca spp, Inulin oder Farnesol ausgewählt ist.

2. Extrudierte, wasserlösliche Folie nach Anspruch 1, die durch Extrusion bei einer Temperatur von 90 °C bis 125 °C erhalten werden kann, wobei das Polymer eine amorphe Polyvinylalkohol-Matrix ist.

3. Extrudierte, wasserlösliche Folie nach Anspruch 1, ferner umfassend bis zu 50 Gewichts-% Stärke.

4. Extrudierte, wasserlösliche Folie nach Anspruch 1, ferner umfassend bis zu 30 Gewichts-% Ethylenvinylacetat (EVA) .

5. Extrudierte, wasserlösliche Folie nach Anspruch 1, wobei die Folie eine Wasserauflösungsgeschwindigkeit von 5 Sekunden bis 30 Minuten hat, wie durch eine Wasserauflösungsprüfung der vorliegenden Offenbarung ermittelt, oder
wobei die Folie ein Flächengewicht von 5 g/m² bis 500 g/m² hat; oder wobei die Folie eine Zugfestigkeit von 0,5 MPa bis 50 MPa gemäß einer Zugprüfung der vorliegenden Offenbarung hat; oder wobei die Folie eine Verlängerung von 2 % bis 200 % gemäß einer Zugprüfung der vorliegenden Offenbarung hat.

6. Saugfähiger Körperpflegeartikel, umfassend: ein saugfähiges Element, das zwischen einer wasserundurchlässigen Außenschicht und einer wasserdurchlässigen Auskleidung angeordnet ist, wobei die Auskleidung eine zum Körper weisende Fläche und eine gegenüberliegende zur Kleidung weisende Fläche hat; und wobei die extrudierte, wasserlösliche Folie nach Anspruch 1 an der Auskleidung oder einer Fläche des saugfähigen Elements neben der Auskleidung angebracht ist.

7. Verfahren zur Ausbildung einer wasserlöslichen Folie, das Verfahren umfassend:
Ausbilden eines homogenen Gemischs, das ein wasserlösliches Polymer, das Polyvinylalkohol ist, und mindestens einen Wirkstoff umfasst; und
Extrudieren des homogenen Gemischs, um eine Folie bei einer Temperatur von 50 °C bis 150 °C auszubilden;
wobei der Wirkstoff aus Yucca spp, Inulin oder Farnesol ausgewählt ist.

8. Verfahren nach Anspruch 7, wobei das Polymer ein amorpher Polyvinylalkohol ist.

9. Verfahren nach Anspruch 7, wobei das Polymer eine amorphe Polyvinylalkohol-Matrix ist und wobei das homogene Gemisch bei einer Temperatur von 90 °C bis 125 °C extrudiert wird.

10. Verfahren nach Anspruch 7, wobei das homogene Gemisch bei einer Temperatur von 50 °C bis 125 °C extrudiert wird.

11. Verfahren nach Anspruch 7, wobei das homogene Gemisch bei einer Temperatur von 90 °C bis 125 °C extrudiert wird.

## Revendications

1. Film extrudé soluble dans l'eau obtenu par extrusion à une température de 50 à 150 °C à partir d'un matériau homogène comprenant un polymère soluble dans l'eau qui est de l'alcool polyvinylique et entre 0,1 % à 50 % en poids du film d'agent actif, où l'agent actif est choisi parmi le Yucca spp, l'inuline ou le farnésol.

2. Film extrudé soluble dans l'eau selon la revendication 1, obtenu par extrusion à une température de 90 °C à 125 °C, où le polymère est une matrice d'alcool polyvinylique amorphe.

3. Film extrudé soluble dans l'eau selon la revendication 1, comprenant en outre jusqu'à 50 % d'amidon en poids.

4. Film extrudé soluble dans l'eau selon la revendication 1, comprenant en outre jusqu'à 30 % en poids d'éthylène acétate de vinyle (EVA).

5. Film extrudé soluble dans l'eau selon la revendication 1, où le film a une vitesse de dissolution dans l'eau de 5 secondes à 30 minutes, telle que déterminée par un test de dissolution dans l'eau de la présente divulgation, ou
où le film a un grammage de 5 gsm à 500 gsm ; ou où le film a une résistance à la traction de 0,5 MPa à 50 MPa selon un test de traction de la présente divulgation ; ou où le film a une élongation de 2 % à 200 % selon un test de traction de la présente divulgation.

6. Article absorbant personnel comprenant : un élément absorbant disposé entre feuillet arrière imperméable à l'eau et une doublure perméable à l'eau, où la doublure a une surface faisant face au corps et une surface faisant face au vêtement opposée ; et où le film extrudé soluble dans l'eau selon la revendication 1 est fixé sur la doublure ou une surface de l'élément absorbant adjacent à la doublure.

7. Procédé de formation d'un film soluble dans l'eau, le procédé comprenant :
la formation d'un mélange homogène comprenant un polymère soluble dans l'eau qui est de l'alcool polyvinylique et au moins un agent actif ; et
l'extrusion du mélange homogène afin de former un film à une température de 50 à 150 °C ;
où l'agent actif est choisi parmi le Yucca spp, l'inuline ou le farnésol.

8. Procédé selon la revendication 7, dans lequel le polymère est un alcool polyvinylique amorphe.

9. Procédé selon la revendication 7, dans lequel le polymère est une matrice d'alcool polyvinylique amorphe et dans lequel le mélange homogène est extrudé à une température de 90 °C à 125 °C.

10. Procédé selon la revendication 7, dans lequel le mélange homogène est extrudé à une température de 50 °C à 125 °C.

11. Procédé selon la revendication 7, dans lequel le mélange homogène est extrudé à une température de 90 °C à 125 °C.
